# EUROPÄISCHE PATENTSCHRIFT

(11) **EP 2 612 153 B1**
(45) Veröffentlichungstag und Bekanntmachung des Hinweises auf die Patenterteilung: **28.11.2018**
(21) Anmeldenummer: 11763867.6
(22) Anmeldetag: 01.09.2011
(51) Int. Cl.: G01N 35/00

(54) **VORRICHTUNG UND VERFAHREN ZUR ANALYSE BIOLOGISCHER PROBEN**
APPARATUS AND METHOD FOR ANALYSING BIOLOGICAL SAMPLES
DISPOSITIF ET PROCÉDÉ D'ANALYSE D'ÉCHANTILLONS BIOLOGIQUES

(30) Priorität: 03.09.2010 DE 102010044283
(43) Veröffentlichungstag der Anmeldung: 10.07.2013
(73) Patentinhaber: M04 Tech UG (Haftungsbeschränkt), 39118 Magdeburg (DE)
(72) Erfinder: POMMER, Ansgar J., 91054 Erlangen (DE); GANZ, Michael, 39118 Magdeburg (DE); BARSCHEL, Eike, 39118 Magdeburg (DE); PHILIPSEN, Lars, 39114 Magdeburg (DE)
(74) Vertreter: Carlsohn, Alexander
(86) Internationale Anmeldenummer: PCT/EP2011/065156
(87) Internationale Veröffentlichungsnummer: WO 2012/028699

(56) Entgegenhaltungen:
- WO-A2-2010/011931
- US-A1- 2003 000 597
- US-A1- 2005 170 356

## Beschreibung

Die Erfindung betrifft eine Vorrichtung zur Analyse biologischer Proben, die ein Kit, umfassend die für die Analyse erforderlichen Marker aufweist, und ein Verfahren zur Analyse der biologischen Proben mittels der Vorrichtung und des Kits.

Aus dem Stand der Technik sind Vorrichtungen und damit verbundene Verfahren zur Detektion von Proteinen in Zellproben bekannt. Dabei werden die Proben mit Markern versetzt, die einen Bindungspartner mit hoher Affinität zu einem der zu detektierenden Proteine und ein Chromophor, beispielweise ein Fluorophor, aufweisen, dessen emittiertes Licht mit Hilfe eines Mikroskops, beispielsweise eines Widefield-Fluoreszenzmikroskops erfasst werden kann. Auf diese Weise können Fluoreszenbilder der Probe erhalten werden, deren Färbung für die dort nachzuweisenden Proteine charakteristisch ist. Die Bilddaten werden in einer Datenbank gespeichert. Anschließend können die aus der Datenbank ausgelesenen Bilddaten mittels einer Analysesoftware bearbeitet und visualisiert werden. Auf diese Weise kann beispielsweise die Größe des Zellkerns einer Zelle gemessen werden.

Stellt sich nun die Aufgabe, die Größe der Zellkerne von Zellen einer biologischen Probe in der Praxis zu bestimmen, so hat der Anwender zunächst die Marker auszuwählen, die zur Lösung dieser Aufgabe erforderlich sind. Anschließend müssen die Messprotokolle bestimmt werden, die erforderlich sind, um die gestellte Aufgabe unter Verwendung der ausgewählten Marker zu lösen. Diese Messprotokolle hat er einerseits an den entsprechenden Geräten, beispielsweise dem Fluoreszenzmikroskop, einzustellen, andererseits muss für das Ineinandergreifen der einzelnen Elemente eines Messprotokolls, die Handlungen betreffen, die an verschiedenen Geräten vorgenommen werden müssen, Sorge getragen werden. Erhält der Anwender dann mittels des Fluoreszenzmikroskops die gewünschten Bilddaten, so muss er die Analysesoftware so konfigurieren, dass er die von ihm gewünschten Informationen, d.h. die Angaben zur Größe der Zellkerne, den Bilddaten tatsächlich entnehmen kann. Dazu muss er geeignete Softwareroutinen auswählen und deren Eignung anhand der Messprotokolle und des visualisierten Ergebnisses dieser Verfahrensweise überprüfen.

Jede der Maßnahmen, die der Anwender treffen muss, um von seiner Analyseaufgabe bis zum Analyseergebnis zu gelangen, ist zeitaufwendig und fehleranfällig. Dabei können sich einzelne Fehler potenzieren, was falsche Analyseergebnisse oder ein Versagen der Analysemethode insgesamt zur Folge haben kann.

US 2005/170356 A1 offenbart eine Mehrreagenzien-Packung, die einen Lese-Schreibe-Speicherchip mit Daten aufweist. Die Daten können zwischen der Packung und einem Analysegerät ausgetauscht werden.

US 2003/000597 A1 offenbart eine Vorrichtung zur automatisierten Lagerung von Trays. Die Vorrichtung weist ein Mikroskop auf, dessen Daten an einen Klassifizierungsprozessor übertragen und dort klassifiziert werden.

Aufgabe der Erfindung ist es, die Nachteile nach dem Stand der Technik zu beseitigen. Es soll insbesondere eine Vorrichtung zur Analyse biologischer Proben angegeben werden, die eine Automatisierung der Analyse biologischer Proben ermöglicht und ein Kit, das die für die Analyse erforderlichen Marker umfasst, aufweist. Es soll ferner ein Verfahren zur Analyse der biologischen Proben mittels der Vorrichtung und des Kits angegeben werden.

Diese Aufgabe wird durch die Merkmale der Ansprüche 1 und 5 gelöst. Zweckmäßige Ausgestaltungen der Erfindungen ergeben sich aus den Merkmalen der Unteransprüche.

Nach Maßgabe der Erfindung ist eine Analysevorrichtung zur automatisierten Analyse einer biologischen Probe vorgesehen, umfassend
- das Kit, umfassend eine Einheit (Aufnahmeeinheit) mit einer Vielzahl von Reservoirs zur Aufnahme eines oder mehrerer Marker, die zur Analyse der biologischen Probe erforderlich sind, sowie, falls erforderlich, der dazu benötigten Betriebsstoffe, sowie eine Einheit (Speichereinheit) zur elektronischen Speicherung eines oder mehrerer elektronisch gespeicherter Messprotokolle, umfassend Instruktionen für die Durchführung der Analyse der biologischen Probe mittels der Marker, wobei die Speichereinheit in die Aufnahmeeinheit integriert ist und wobei die Aufnahmeeinheit des Kits als ein Mikrofluidiksystem realisiert ist;
- eine Steuereinrichtung, umfassend
   - eine Datenverarbeitungseinheit;
   - eine Einheit zur Aufnahme des Kits;
   - eine Schnittstelle, die die Kommunikation zwischen der Datenverarbeitungseinheit und dem Kit ermöglicht;
   - eine Schnittstelle, die die Kommunikation zwischen der Datenverarbeitungseinheit und einer Transfereinheit zum Transfer der Marker und, falls vorhanden, der Betriebsstoffe von dem Kit zu einer Detektionseinheit ermöglicht;
   - eine Schnittstelle, die die Kommunikation zwischen der Datenverarbeitungseinheit und der Detektionseinheit zur Erzeugung von Bilddaten von der biologischen Probe ermöglicht; und
   - eine Schnittstelle, die die Kommunikation zwischen der Datenverarbeitungseinheit und einer Analyseeinheit zur Analyse der mittels der Detektionseinheit erhaltenen Bilddaten unter Erzeugung von Analysedaten ermöglicht;
- eine Transfereinheit zum Transfer der Marker von dem Kit zu einer Detektionseinheit, wobei die Transfereinheit über die zugehörige Schnittstelle der Steuereinrichtung mit dieser kommuniziert und die Transfereinheit als ein Mikrofluidiksystem realisiert ist;
- eine Detektionseinheit zur Erzeugung von Bilddaten von der biologischen Probe, wobei die Detektionseinheit über die zugehörige Schnittstelle der Steuereinrichtung mit dieser kommuniziert;
- eine Analyseeinheit zur Analyse der mittels der Detektionseinheit erhaltenen Bilddaten unter Erzeugung von Analysedaten, wobei die Analyseeinheit über die zugehörige Schnittstelle der Steuereinrichtung mit dieser kommuniziert, wobei in der Speichereinheit des Kits
- ein oder mehrere elektronisch gespeicherte Messprotokolle, umfassend Instruktionen für die Durchführung der Analyse der biologischen Probe mittels der Marker; und
- elektronisch gespeicherte Software zur Auswertung von Messergebnissen, die von der biologischen Probe mittels der Marker und unter Verwendung der Messprotokolle erhalten wurden
gespeichert sind.

Das Kit zur Verwendung bei der Analyse einer biologischen Probe kann zumindest
(a) einen oder mehrere Marker, die zur Analyse der biologischen Probe erforderlich sind, sowie, falls erforderlich, die dazu benötigten Betriebsstoffe; und
umfassen.

Das Kit umfasst erfindungsgemäß
(b) ein oder mehrere elektronisch gespeicherte Messprotokolle, umfassend Instruktionen für die Durchführung der Analyse der biologischen Probe mittels der Marker.

Das Kit umfasst zweckmäßigerweise alle Marker, die zur Analyse der biologischen Probe erforderlich sind. Das Kit sollte alle Messprotokolle, die zur Durchführung der Analyse der biologischen Probe erforderlich sind, umfassen.

Ferner umfasst das Kit erfindungsgemäß
(c) elektronisch gespeicherte Software zur Auswertung von Messergebnissen, die von der biologischen Probe mittels der Marker und unter Verwendung der Messprotokolle erhalten wurden.

Überdies kann das Kit umfassen
(d) ein oder mehrere elektronisch gespeicherte Messprotokolle, umfassend Instruktionen für die Präparation der biologischen Probe. Die zur Präparation erforderlichen Betriebsstoffe sind ebenfalls in dem Kit enthalten.

Das Kit umfasst zweckmäßigerweise eine Einheit mit einer Vielzahl von Reservoirs zur Aufnahme der Marker und Betriebsstoffe (die als "Aufnahmeeinheit" bezeichnet wird) sowie eine Einheit zur elektronischen Speicherung der Messprotokolle und optional der Software (die als "Speichereinheit" bezeichnet wird). Die Speichereinheit umfasst vorzugsweise eine Schnittstelle, die die Kommunikation der Speichereinheit mit einer anderen elektronischen Einheit ermöglicht. Vorzugsweise ist die Speichereinheit ein Speichermedium mit einer Hardwareschnittstelle. Beispielsweise kann es sich bei der Speichereinheit um einen Datenspeicher mit USB-Schnittstelle handeln.

Die Speichereinheit ist in die Aufnahmeeinheit integriert. Auf diese Weise wird eine Aufnahmeeinheit mit integrierter Speichereinheit, die eine Schnittstelle aufweist, erhalten.

Die Aufnahmeeinheit des Kits enthält die Marker, die für die Analyse der biologischen Probe gemäß der Messprotokolle, die auf der Speichereinheit elektronisch gespeichert sind, erforderlich sind. Ferner kann die Aufnahmeeinheit die Betriebsstoffe enthalten, die erforderlich sind, um die chemische und/oder physikalische Anbindung der Marker an die nachzuweisenden Bestandteile der biologischen Probe bewirken zu können. Die einzelnen Marker und, falls vorhanden, die einzelnen Betriebsstoffe sind in gesonderten Reservoirs der Aufnahmeeinheit untergebracht. Um die Haltbarkeitsdauer eines Kits zu erhöhen, sind die Marker und, falls vorhanden, die Betriebsstoffe, beispielsweise in lyophilisierter Form in den Reservoirs enthalten. Die Reservoirs sind zweckmäßigerweise verschlossen, beispielsweise mit einer Folie, die unmittelbar vor dem Einsatz des Kits entfernt wird.

Die Aufnahmeeinheit des Kits ist als ein Mikrofluidiksystem realisiert.

Das Kit besteht somit vorzugsweise aus einem mechanischen Teil, der Aufnahmeeinheit, in dem Marker und Betriebsstoffe gelagert sind, und einem elektronischen Speichermedium, der Speichereinheit, in dem alle für die Durchführung des Analyse notwendigen Informationen bereitgestellt werden. Diese Informationen sind in der vorliegenden Erfindung als Messprotokolle bezeichnet.

Der Begriff "Marker" beschreibt in der vorliegenden Erfindung eine chemische Verbindung, die mit einer chemischen oder biologischen Einheit, beispielsweise einem Protein, das in der biologischen Probe enthalten ist, reagieren kann. Die Anbindung des Markers an die chemische oder biologische Einheit kann mittels analytischer Verfahren nachgewiesen werden. Ein Marker kann beispielsweise einen Bindungspartner mit hoher Affinität zu einem der zu detektierenden Proteine und zum anderen ein Chromophor, beispielweise ein Fluorophor, aufweisen. Der Bindungspartner und das Chromophor können über einen Linker miteinander verbunden sein. Der Bindungspartner kann beispielsweise ein Chelator, insbesondere wenn Ionen in der biologischen Probe nachgewiesen werden sollen, oder ein Antikörper sein, wenn Antigene in der biologischen Probe nachgewiesen werden sollen. Wird die biologische Probe mit dem Marker versetzt, so kann die Anbindung des Markers an die chemische und biologische Einheit qualitativ oder quantitativ nachgewiesen werden, indem das Chromophor zur Lichtemission angeregt wird. Das emittierte Licht kann dann erfasst werden, beispielsweise mittels eines Mikroskops wie eines Widefield-Fluoreszenzmikroskops.

Der Begriff "Betriebsstoff" beschreibt in der vorliegenden Erfindung eine chemische Substanz, die erforderlich ist, um die Marker, die in dem Kit enthalten sind, mit der biologischen Probe in der in den Messprotokollen vorgeschriebenen Weise in Kontakt und/oder in Reaktion bringen zu können. Beispiele für Betriebsstoffe sind Lösungsmittel wie Wasser oder Isopropanol. Umfasst das Kit auch Messprotokolle mit Instruktionen zur Präparation der biologischen Probe, so umfasst der Begriff "Betriebsstoffe" auch die dafür erforderlichen chemischen Substanzen.

Der Begriff "Messprotokoll" beschreibt in der vorliegenden Erfindung die Summe aus Informationen und Anweisungen, die erforderlich sind, um die biologische Probe mittels der Marker unter Verwendung einer Analysevorrichtung analysieren zu können. Ein Messprotokoll umfasst beispielsweise
- Informationen über die Art und Menge der Marker, die in dem Kit enthalten sind;
- Informationen über die Art und Menge der Betriebsstoffe, die in dem Kit enthalten sind;
- Informationen über das Reservoir, in dem sich der jeweilige Marker oder Betriebsstoff aus der Aufnahmeeinheit befindet;
- Anweisungen zu den relativen Zeitpunkten für die Entnahme jedes Markers oder Betriebsstoffes aus dem jeweiligen Reservoir der Aufnahmeeinheit, bezogen auf den Start des Analyseverfahrens;
- Anweisungen zu dem Ort, zu dem die aus der Aufnahmeeinheit entnommenen Marker oder Betriebsstoffe überführt werden sollen;
- Anweisungen zu Verweildauer der Marker oder Betriebsstoffe an diesen Ort Marker oder Betriebsstoffe;
- Anweisungen zu den Grundeinstellungen der einzelnen Komponenten der Analysevorrichtung beim Start des Analyseverfahrens;
- Anweisungen zur Steuerung der einzelnen Komponenten der Analysevorrichtung nach dem Start des Analyseverfahrens, beispielsweise nach dem Empfang bestimmter Marker, Betriebsstoffe oder Daten;
- Anweisungen, betreffend die Art der zu detektierenden Bilddaten;
- Anweisungen, betreffend die Art der durchzuführenden Analyse;
- Anweisungen, betreffend den zeitlichen Ablauf der einzelnen Schritte des Analyseverfahrens.

Bei dem Ort kann es sich beispielsweise um die Probenaufnahme der Detektionseinheit handeln.

Umfasst das Kit auch Messprotokolle mit Instruktionen zur Präparation der biologischen Probe, so umfasst der Begriff "Messprotokoll" in der vorliegenden Erfindung zusätzlich die Summe aus Informationen und Anweisungen, die erforderlich sind, um Präparation der biologischen Probe mittels der dazu im Kit enthaltenen Betriebsstoffe unter Verwendung der Analysevorrichtung durchführen zu können.

In einer bevorzugten Ausführungsform sind eine oder mehrere der folgenden Informationen in der Speichereinheit des Kits gespeichert:
- Vorschriften zur Applikation der Marker und Betriebsstoffe auf die Probe (Angaben zur Reihenfolge, Konzentration, Einwirkdauer, Vor- und Nachbehandlung, Kalibrierung usw.),
- Vorschriften zur Steuerung der Detektionseinheit (Mikroskop oder anderes),
- Vorschriften zur Durchführung der eigentlichen Messung (Bildaufnahme mit Parametern wie Belichtungszeit, erforderliche Wellenlängen, Filter, Aufnahmepositionen etc.),
- Vorschriften zur adaptiven Beeinflussung der obengenannten Parameter im Sinne der Optimierung und Qualitätssicherung während des Messprozesses,
- Operatoren zur Abarbeitung der obengenannten Vorschriften
- Operatoren zur Steuerung peripherer Geräte,
- numerische Operatoren zur Auswertung, Analyse und Darstellung der Messdaten und daraus gewonnener Analyseergebnisse,
- Software und Software-Updates für Steuereinrichtung.

Die Steuereinrichtung zur Steuerung der Analyse einer biologischen Probe umfasst die Datenverarbeitungseinheit und die Einheit zur Aufnahme des Kits. Die Steuereinrichtung umfasst ferner
- die Schnittstelle, die die Kommunikation zwischen der Datenverarbeitungseinheit und dem Kit ermöglicht;
- die Schnittstelle, die die Kommunikation zwischen der Datenverarbeitungseinheit und der Transfereinheit zum Transfer der Marker und, falls vorhanden, der Betriebsstoffe von dem Kit zu einer Detektionseinheit ermöglicht;
- die Schnittstelle, die die Kommunikation zwischen der Datenverarbeitungseinheit und der Detektionseinheit zur Erzeugung von Bilddaten von der biologischen Probe ermöglicht; und
- die Schnittstelle, die die Kommunikation zwischen der Datenverarbeitungseinheit und der Analyseeinheit zur Analyse der mittels der Detektionseinheit erhaltenen Bilddaten unter Erzeugung von Analysedaten ermöglicht.

Vorzugsweise umfasst die Steuereinrichtung überdies eine oder mehrere der folgenden Schnittstellen:
- eine Schnittstelle, die die Kommunikation zwischen der Datenverarbeitungseinheit und einer Datenbank zum Speichern von Bilddaten und/oder Analysedaten ermöglicht; und
- eine Schnittstelle, die die Kommunikation zwischen der Datenverarbeitungseinheit und einer Visualisierungseinheit zur Visualisierung von Bilddaten und/oder Analysedaten ermöglicht.

Vorzugsweise umfasst die Datenverarbeitungseinheit zumindest einen Mikroprozessor, Datenspeicher und Eingabe- und Ausgabeeinheiten. Die Datenverarbeitungseinheit ist vorzugsweise mit einem Betriebssystem ausgestattet, das die vom Kit bereitgestellten Messprotokolle wie beispielsweise Operatoren und Vorschriften lädt und ausführt. Das Betriebssystem verwaltet die zur Verfügung stehende Hardware der Analysevorrichtung und startet auf dieser Hardware die für das Analyseverfahren notwendige Prozesse. Es ist mit dem Betriebssystem eines Computers vergleichbar.

Die Analyseeinheit kann mittels der Datenverarbeitungseinheit der Steuereinrichtung oder einer gesonderten Datenverarbeitungseinrichtung realisiert werden.

Die Einheit zur Aufnahme eines Kits ermöglicht es dem Anwender, ein von ihm ausgewähltes Kit, dass für die Analyse einer biologischen Probe geeignet ist, in die Steuereinrichtung einzusetzen. Dabei wird über die Schnittstelle des Kits einerseits und die Schnittstelle der Steuereinrichtung, die die Kommunikation zwischen der Datenverarbeitungseinheit und dem Kit ermöglicht, eine Verbindung hergestellt, über die die Messprotokolle von der Speichereinheit des Kits auf die Datenverarbeitungseinheit der Steuereinrichtung übertragen werden können. Damit ist sichergestellt, dass die Marker und, falls vorhanden, die Betriebsstoffe des Kits, bestimmungsgemäß eingesetzt werden, was Analysefehler vermeidet, eine Automatisierung der Analyse biologischer Proben ermöglicht, und eine zeitliche und räumliche Verringerung des Analyseaufwandes bedeutet.

Die Schnittstelle der Speichereinheit des Kits und die Schnittstelle der Steuereinrichtung, die die Kommunikation zwischen der Datenverarbeitungseinheit und dem Kit ermöglicht, sind aufeinander abgestimmt. Handelt es sich bei der Schnittstelle der Speichereinheit des Kits um eine USB-Schnittstelle, so ist die zugehörige Schnittstelle der Steuereinrichtung, d. h. die Schnittstelle der Steuereinrichtung, die die Kommunikation zwischen der Datenverarbeitungseinheit und dem Kit ermöglicht, ebenfalls eine USB-Schnittstelle. Beispielsweise kann die Speichereinheit des Kits einen USB-Stecker, die Steuereinrichtung eine USB-Buchse aufweisen. Beim Einsetzen des Kits in die Einrichtung der Steuereinrichtung zur Aufnahme des Kits wird gleichzeitig der USB-Stecker in die USB-Buchse eingeführt. Damit ist die Kommunikationsverbindung zwischen dem Kit und der Steuereinrichtung hergestellt. Außerdem befinden sich die Reservoirs des Kits in einer vorbestimmten Position in der Steuereinrichtung.

Die übrigen Schnittstellen der Steuereinrichtung dienen zur Kommunikation mit Einrichtungen, die zur Analyse der biologischen Probe erforderlich sind. Diese Einrichtungen umfassen folgende Einrichtungen:
- die Transfereinheit zum Transfer von Markern und, falls vorhanden, Betriebsstoffen von dem Kit zu der Detektionseinheit;
- die Detektionseinheit zur Erzeugung von Bilddaten von der biologischen Probe; und
- die Analyseeinheit zur Analyse der mittels der Detektionseinheit erhaltenen Bilddaten unter Erzeugung von Analysedaten.

Ferner können die Einrichtungen, die zur Analyse der biologischen Probe erforderlich sind, eine oder mehrere der folgenden Einrichtungen umfassen:
- eine Datenbank zum Speichern von Bilddaten und/oder Analysedaten; und
- eine Visualisierungseinheit zur Visualisierung von Bilddaten und/oder Analysedaten.

Die Schnittstelle der Steuereinrichtung, die die Kommunikation mit einer dieser Komponenten ermöglicht, wird im Folgenden auch als "zugehörige Schnittstelle" bezeichnet. Beispielsweise ist die Schnittstelle, die die Kommunikation zwischen der Datenverarbeitungseinheit und einer Transfereinheit ermöglicht, die zugehörige Schnittstelle zur Kommunikation zwischen der Datenverarbeitungseinheit der Steuereinrichtung und der Transfereinheit.

Die Steuereinrichtung kann als separates Gerät ausgebildet sein und so eine eigenständige Vorrichtung darstellen. Ist die Steuereinrichtung ein separates Gerät, so wird sie im Folgenden als Steuergerät bezeichnet.

Der Transfer der Marker und/oder Betriebsstoffe wird mit einem Mikrofluidiksystem durchgeführt.

Die Analysevorrichtung umfasst:
- die Transfereinheit zum Transfer der Marker von dem Kit zu einer Detektionseinheit, wobei die Transfereinheit über die zugehörige Schnittstelle der Steuereinrichtung mit dieser kommuniziert;
- die Detektionseinheit zur Erzeugung von Bilddaten von der biologischen Probe, wobei die Detektionseinheit über die zugehörige Schnittstelle der Steuereinrichtung mit dieser kommuniziert; und
- die Analyseeinheit zur Analyse der mittels der Detektionseinheit erhaltenen Bilddaten unter Erzeugung von Analysedaten, wobei die Analyseeinheit über die zugehörige Schnittstelle der Steuereinrichtung mit dieser kommuniziert.

Die Analysevorrichtung kann ferner zumindest eine der folgenden Einheiten umfassen:
- eine Datenbank zum Speichern von Bilddaten und/oder Analysedaten, wobei die Datenbank über die zugehörige Schnittstelle der Steuereinrichtung mit dieser kommuniziert; und
- eine Visualisierungseinheit zur Visualisierung von Bilddaten und/oder Analysedaten, wobei die Visualisierungseinheit über die zugehörige Schnittstelle der Steuereinrichtung mit dieser kommuniziert.

Die erfindungsgemäße Analysevorrichtung ermöglicht die automatisierte, rasche und fehlerarme Ausführung der Analyse biologischer Proben.

Nach Maßgabe der Erfindung ist überdies ein Verfahren zur automatisierten Analyse einer biologischen Probe mittels einer erfindungsgemäßen Analysevorrichtung vorgesehen, das die folgenden Schritte umfasst:
(a) Bereitstellen eines Kits, umfassend die zur Analyse einer vorgegebenen biologischen Probe erforderlichen Marker, Betriebsstoffe, Messprotokolle und optional Software;
(b) Einführen des Kits in die Steuereinrichtung und Übertragen von dort gespeicherten Messprotokollen in die Datenverarbeitungseinheit der Steuereinrichtung;
(c) Bestimmen der Anweisungen aus den Messprotokollen, die zur Steuerung der Transfereinheit erforderlich sind, mittels der Datenverarbeitungseinheit und Übertragen dieser Anweisungen über die zugehörige Schnittstelle der Steuereinrichtung auf die Transfereinheit;
(d) Bestimmen der Anweisungen aus den Messprotokollen, die zur Steuerung der Detektionseinheit erforderlich sind, mittels der Datenverarbeitungseinheit und Übertragen dieser Anweisungen über die zugehörige Schnittstelle der Steuereinrichtung auf die Detektionseinheit;
(e) Transfer von Markern und Betriebsstoffen von dem Kit zu der Detektionseinheit mittels der Transfereinheit anhand der in Schritt (c) übertragenen Anweisungen;
(f) Erzeugen von Bilddaten der biologischen Probe mittels der Detektionseinheit anhand der in Schritt (d) übertragenen Anweisungen;
(g) Bestimmen der Anweisungen aus den Messprotokollen, die zur Steuerung der Analyseeinheit erforderlich sind, mittels der Datenverarbeitungseinheit und Übertragen dieser Anweisungen über die zugehörige Schnittstelle der Steuereinrichtung zu der Analyseeinheit, wobei Software, die vom Kit in die Datenverarbeitungseinheit übertragen worden ist, von der Datenverarbeitungseinheit zu der Analyseeinheit übertragen wird; und
(h) Transfer der in Schritt (f) erhaltenen Bilddaten zu der Analyseeinheit und Analysieren der Bilddaten mittels der Analyseeinheit unter Verwendung der in Schritt (e) übertragenen Anweisungen und der in Schritt (g) übertragenen Software, wodurch Analysedaten erhalten werden.

Es ist vorgesehen, dass in Schritt (g) ferner Software, die vom Kit in die Datenverarbeitungseinheit übertragen worden ist, von der Datenverarbeitungseinheit zu der Analyseeinheit übertragen wird und dass in Schritt (h) zusätzlich zu den Anweisungen die Software zum Analysieren der Bilddaten verwendet wird. Die Software kann bestimmte Algorithmen umfassen, die in der Analyseeinheit ausgeführt werden, um aus den Bilddaten Informationen über die biologische Probe gewinnen zu können.

Umfasst die Analysevorrichtung eine Datenbank, so können die Bilddaten und/oder die Analysedaten zu der Datenverarbeitungseinheit übertragen und dort in einer Datenbank gespeichert werden.

Umfasst die Analysevorrichtung eine Visualisierungseinheit, so können die Bilddaten und/oder Analysedaten von der Datenverarbeitungseinheit über deren zugehörige Schnittstelle zu einer Visualisierungseinheit übertragen werden.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine schematische Darstellung einer Ausführungsform der erfindungsgemäßen Analysevorrichtung mit eingesetztem Kit; und

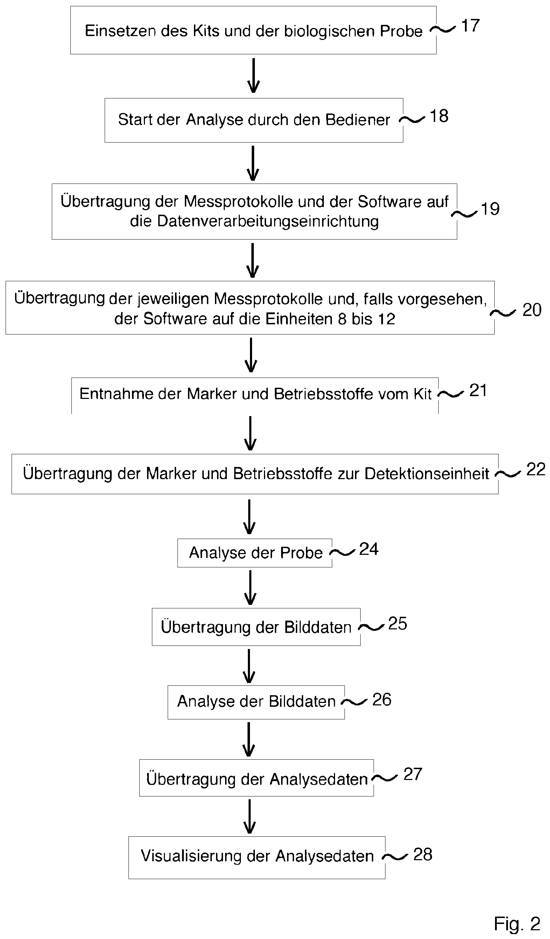

- Fig. 2: ein Ablaufdiagramm, das den Ablauf einer Ausführungsform des erfindungsgemäßen Verfahrens mittels der in Fig. 1 gezeigten Analysevorrichtung darstellt.

### Beispiel 1: Kit

Nachstehend wird ein Kit beschrieben, das zur Bestimmung der Größe des Zellkerns und der Größe der Zelle mononuklearer Zellen aus dem Blut und zur Berechnung des Größenverhältnisses von Zellkern zu Zelle eingesetzt werden kann. Das Kit umfasst:
(a) Marker und Betriebsstoffe:
   - *Marker 1 (Detektion des Zellkerns)*: *Propidiumiodid*
      - Anregungswellenlänge 536 nm,
      - Emissionswellenlänge 617 nm,
      - Konzentration: 0,1 µg/ml
      - Inkubationszeit: 15 min
   - *Marker 2 (Detektion der Zellmembran): anti- CD45 Antikörper direkt gekoppelt mit Alexa 488 Farbstoff*
      - Anregungswellenlänge 495 nm,
      - Emissionswellenlänge 519 nm,
      - Konzentration: 1 µg/ml
      - Inkubationszeit: 15 min
   - *Waschlösung*
      - PBS (phosphate buffered saline) mit 0,5%BSA (Bovines Serumalbumin)
(b) elektronisch gespeicherte Messprotokolle:
   - eine Vorschrift zur Herstellung des Präparates (Probe) für die Mikroskopie
   - eine Vorschrift zur Applikation der Marker auf die biologische Probe
   - eine Vorschrift zur digitalen Bildaufnahme am Widefield-FluoreszenzMikroskop, inklusive der Aufnahme von Kalibrierungsbildern
(c) elektronisch gespeicherte Software zur Auswertung der Messergebnisse:
   - Software mit Algorithmen zur Registrierung und Bildnormierung bzw. Bildkorrektur
   - Software mit Algorithmen zur Bestimmung der markierten Zellmembran
   - Software mit Algorithmen zur Bestimmung des markierten Zellkerns
   - Software mit Algorithmen zur Bestimmung des Verhältnisses der Größe des Zellkerns und der gesamten Zelle
   - Software mit Algorithmen zur Statistische Auswertung und Visualisierung

### Beispiel 2: Analysevorrichtung

Nachstehend wird eine Analysevorrichtung beschrieben, die zur Bestimmung der Größe des Zellkerns und der Größe der Zelle mononuklearer Zellen aus dem Blut und zur Berechnung des Größenverhältnisses von Zellkern zu Zelle eingesetzt werden kann. Dazu umfasst die Analysevorrichtung 1 eine Steuereinrichtung 3 sowie eine Transfereinheit 8, eine Detektionseinheit 9, eine Analyseeinheit 10, eine Visualisierungseinheit 11 sowie eine Datenbank 12 zum Speichern von Bilddaten und Analysedaten. Die Steuereinrichtung 3 umfasst eine Datenverarbeitungseinheit 13 und eine Einheit (nicht gezeigt) zur Aufnahme eines Kits 2.

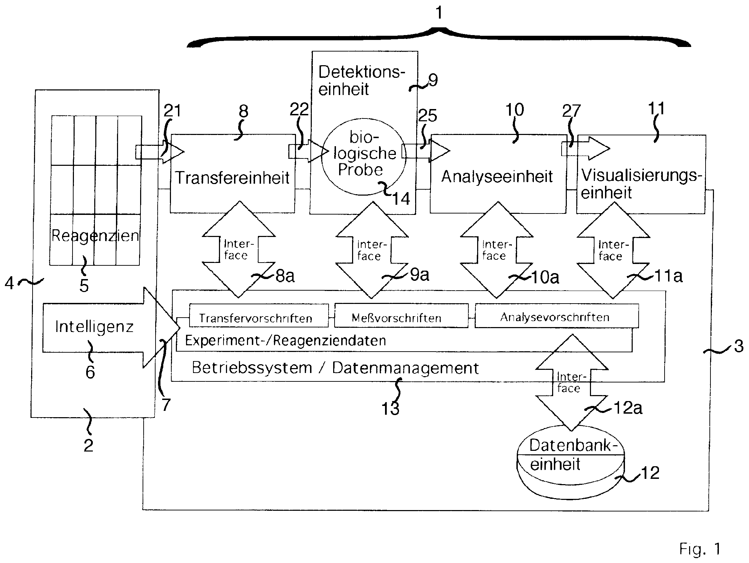

Das Kit 2 umfasst eine Trägerplatte 4, auf der eine Vielzahl von Reservoirs 5 zur Aufnahme von Markern und Betriebsstoffen vorgesehen ist. Auf der Trägerplatte 5 ist ferner eine Speichereinheit 6 zur Speicherung von Messprotokollen und Software vorgesehen. Die Speichereinheit 6 verfügt über eine Schnittstelle 7 (in Fig. 1 in Form eines Pfeils gezeigt), die in Kontakt mit der Steuereinrichtung 3 gebracht werden kann.

In Fig. 1 ist das Kit 2 in die Aufnahmeeinheit der Steuereinrichtung 3 eingesetzt 17. Damit steht die Speichereinheit 6 des Kits 2 über deren Schnittstelle 7 in Kontakt mit der Datenverarbeitungseinheit 13 der Steuereinrichtung 3. Gleichzeitig hat die Transfereinheit 8 der Analysevorrichtung 1 Zugriff auf die Marker und Betriebsstoffe, die sich in den Reservoirs 5 des Kits 2 befinden.

Die Steuereinrichtung 3 weist Schnittstellen auf, über die sie mit den Einheiten 8 bis 12 sowie mit der Speichereinheit 6 des Kits 2 kommunizieren kann. Nach dem Einsetzen des Kits 2 in die Aufnahmeeinheit der Steuereinrichtung 3 und das Einbringen einer biologischen Probe 14 in die Detektionseinheit 9 kann das Analyseverfahren von einem Bediener gestartet werden. Nach dem Start 18 werden die Messprotokolle, die Software und Experiment- und Reagenziendaten, die in der Speichereinheit 6 gespeichert sind, von der Datenverarbeitungseinheit 13 der Steuereinrichtung 3 abgerufen 19. Anschließend werden die Messprotokolle und die Software von der Steuereinrichtung 3 auf die zugehörigen Einheiten 8 bis 11 der Analysevorrichtung 1 übertragen 20. Dabei werden
(i) die Messprotokolle (in Fig. 1 als Transfervorschriften bezeichnet), die den Transfer der Marker und Betriebsstoffe zur Probe und auch die Verweildauer der Marker und Betriebsstoffe auf der Probe betreffen, über die Schnittstelle 8a zwischen der Steuereinrichtung 3 und der Transfereinheit 8 zu der Transfereinheit 8 übertragen;
(ii) die Messprotokolle (in Fig. 1 als Messvorschriften bezeichnet), die die Durchführung des Analyseverfahrens, d. h. das Versetzen der biologischen Probe mit den Markern und den erforderlichen Betriebsstoffen und die Erzeugung der Bilddaten, betreffen, über die Schnittstelle 9a zwischen der Steuereinrichtung 3 und der Detektionseinheit 9 zu der Detektionseinheit 9 übertragen; die Messprotokolle, die Durchführung der Analyse, d. h. die Analyse der Zelle und des Zellkern der Probe, betreffen, über die Schnittstelle 9a zwischen der Steuereinrichtung 3 und der Detektionseinheit 9 zu der Detektionseinheit 9 übertragen;
(iii) die Messprotokolle und die Software (in Fig. 1 als zusammen als Analysevorschriften bezeichnet), die die Analyse der Bilddaten betreffen, z. B. die Algorithmen zur Bestimmung der Größe der Zelle und des Zellkern der Probe, über die Schnittstelle 10a zwischen der Steuereinrichtung 3 und der Analyseeinheit 10 zu der Analyseeinheit 10 übertragen; und
(iv) die Messprotokolle und die Software (in Fig. 1 als zusammen als Analysevorschriften bezeichnet), die die Visualisierung der Analyseergebnisse und/oder der Bilddaten betreffen, über die Schnittstelle 11a zwischen der Steuereinrichtung 3 und der Visualisierungseinheit 11 zu der Visualisierungseinheit 11 übertragen.

Damit verfügen die Einheiten 8 bis 11 über alle erforderlichen Daten, Informationen und Anweisungen, um die Analyse der Probe, einschließlich der Auswertung der Bilddaten und deren Visualisierung automatisch ohne weiteren Eingriff des Bedieners durchführen zu können. Der Ablauf der Analyse der Probe wird von der Datenverarbeitungseinheit 13 der Steuereinheit 3 gesteuert. Dazu werden von der Datenverarbeitungseinheit 13 auf Basis eines Master-Messprotokolls (in Fig. 1 als Experiment- /Reagenziendaten bezeichnet), das ebenfalls auf der Speichereinheit 6 hinterlegt ist und vor dort zu der Datenverarbeitungseinrichtung übertragen wurde, Steuersignale generiert und über die Schnittstellen 8a, 9a, 10a, 11a und 12a zu den Einheiten 8, 9, 10, 11 und 12 übertragen. Ebenso werden von den Einheiten 8 bis 12 Signale erzeugt, die den Empfang des Steuersignals bestätigen und den Vollzug des von den Messprotokollen vorgesehenen Schrittes bestätigen. Die Signale werden von den Einheiten 8, 9, 10, 11 und 12 ebenfalls über die Schnittstellen 8a, 9a, 10a, 11a und 12a übertragen. Ferner dienen die Schnittstellen zur Übertragung von Bilddaten und Analysedaten zwischen den Einheiten 8, 9, 10, 11 und 12.

Zur Generierung der Steuerdaten sowie zum Empfang und zur Verarbeitung der Signale von den Einheiten 8, 9, 10, 11 und 12 weist die Datenverarbeitungseinheit ein übliches Betriebssystem auf, das auch das Datenmanagement ermöglicht.

Auf Basis der empfangenen Messprotokolle und jeweils nach Erhalt des erforderlichen Steuersignals entnimmt 21 die Transfereinheit den vorgegebenen Marker oder den vorgegebenen Betriebsstoffe aus dem vorgegebenen Reservoir 5 des Kits 2 und übertragt 22 den entnommen Marker oder Betriebsstoff zu der Detektionseinheit 9. Der Transfer der Marker und/oder Betriebsstoffe wird mit einem Mikrofluidiksystem durchgeführt.

In der Detektionseinheit 9, die beispielsweise ein Widefield-Fluoreszenzmikroskop ist, wird die biologische Probe 14 gemäß den dorthin übertragenen Messprotokollen analysiert 24, indem die Probe 14 mit den Markern und den erforderlichen Betriebsstoffen in Kontakt gebracht wird und Bilddaten erzeugt werden. Die Bilddaten werden von der Detektionseinheit 9 über die Schnittstelle 9a, die Datenverarbeitungseinrichtung 13 und die Schnittstelle 10a zu der Analyseeinheit 10 übertragen 25 und dort gemäß den Messprotokollen und der Software, die zur Analyseeinheit 10 übertragen worden sind, analysiert 26. Die Analysedaten werden schließlich von der Analyseeinheit 10 über die Schnittstelle 10a, die Datenverarbeitungseinrichtung 13 und die Schnittstelle 11a zu der Visualisierungseinheit 11 übertragen 27 und können dort vom Bediener betrachtet werden 28. Ebenfalls können die Bilddaten von der Datenverarbeitungseinrichtung 13 zu der Visualisierungseinheit 11 übertragen werden. Die Visualisierungseinheit kann ein Bildschirm oder ein Display sein.

Ferner können die Bilddaten und Analysedaten von der Datenverarbeitungseinrichtung 13 über Schnittstelle 12a zu einer Datenbank 12 überführt werden. Von dort können die Bilddaten und Analysedaten jederzeit auf Wunsch eines Bedieners wieder zur Visualisierungseinheit 11 über Schnittstelle 12a, Datenverarbeitungseinheit 13 und Schnittstelle 11a übertragen werden.

Nach Abarbeitung aller Messprotokolle können das Kit 2 aus der Aufnahmeeinheit der Steuereinrichtung 3 und, falls keine weitere Analyse derselben Probe gewünscht ist, die analysierte Probe 14 aus der Detektionseinheit 9 entnommen werden. Mit Einsatz eines neuen Kits 2' in die Aufnahmeeinheit der Steuereinrichtung 3 kann eine weitere Analyse derselben oder einer anderen biologischen Probe 14 gestartet werden.

### Bezugszeichenliste

- 1: Analysevorrichtung
- 2: Kit
- 3: Steuervorrichtung
- 4: Trägerplatte
- 5: Reservoirs
- 6: Speichereinheit
- 7: Schnittstelle
- 8: Transfereinheit,
- 9: Detektionseinheit
- 10: Analyseeinheit
- 11: Visualisierungseinheit
- 12: Datenbank
- 13: Datenverarbeitungseinheit
- 14: biologische Probe

- 17: Einsetzen des Kits und der biologischen Probe
- 18: Start der Analyse durch den Bediener
- 19: Übertragung der Messprotokolle und der Software auf die Datenverarbeitungseinrichtung
- 20: Übertragung der jeweiligen Messprotokolle und, falls vorgesehen, der Software auf die Einheiten 8 bis 12
- 21: Entnahme der Marker und Betriebsstoffe vom Kit
- 22: Übertragung der Marker und Betriebsstoffe zur Detektionseinheit
- 24: Analyse der Probe
- 25: Übertragen der Bilddaten
- 26: Analyse der Bilddaten
- 27: Übertragung der Analysedaten
- 28: Visualisierung der Analysedaten

## Patentansprüche

1. Analysevorrichtung (1) zur automatisierten Analyse einer biologischen Probe (14), umfassend
- ein Kit (2), umfassend eine Einheit (Aufnahmeeinheit) mit einer Vielzahl von Reservoirs (5) zur Aufnahme eines oder mehrerer Marker, die zur Analyse der biologischen Probe (14) erforderlich sind, sowie, falls erforderlich, der dazu benötigten Betriebsstoffe, sowie eine Einheit (Speichereinheit) zur elektronischen Speicherung eines oder mehrerer elektronisch gespeicherter Messprotokolle, umfassend Instruktionen für die Durchführung der Analyse der biologischen Probe (14) mittels der Marker, wobei die Speichereinheit in die Aufnahmeeinheit integriert ist und wobei die Aufnahmeeinheit des Kits (2) als ein Mikrofluidiksystem realisiert ist;
- eine Steuereinrichtung (3), umfassend
- eine Datenverarbeitungseinheit (13);
- eine Einheit zur Aufnahme des Kits (2) ;
- eine Schnittstelle, die die Kommunikation zwischen der Datenverarbeitungseinheit (13) und dem Kit (2) ermöglicht;
- eine Schnittstelle, die die Kommunikation zwischen der Datenverarbeitungseinheit (13) und einer Transfereinheit (8) zum Transfer der Marker und, falls vorhanden, der Betriebsstoffe von dem Kit (2) zu einer Detektionseinheit (9) ermöglicht;
- eine Schnittstelle, die die Kommunikation zwischen der Datenverarbeitungseinheit (13) und der Detektionseinheit (9) zur Erzeugung von Bilddaten von der biologischen Probe (14) ermöglicht; und
- eine Schnittstelle, die die Kommunikation zwischen der Datenverarbeitungseinheit (13) und einer Analyseeinheit (10) zur Analyse der mittels der Detektionseinheit (9) erhaltenen Bilddaten unter Erzeugung von Analysedaten ermöglicht;
- eine Transfereinheit (8) zum Transfer der Marker von dem Kit (2) zu einer Detektionseinheit (9), wobei die Transfereinheit (8) über die zugehörige Schnittstelle der Steuereinrichtung (3) mit dieser kommuniziert und die Transfereinheit (8) als ein Mikrofluidiksystem realisiert ist;
- eine Detektionseinheit (9) zur Erzeugung von Bilddaten von der biologischen Probe (14), wobei die Detektionseinheit (9) über die zugehörige Schnittstelle der Steuereinrichtung (3) mit dieser kommuniziert; und
- eine Analyseeinheit (10) zur Analyse der mittels der Detektionseinheit (9) erhaltenen Bilddaten unter Erzeugung von Analysedaten, wobei die Analyseeinheit (10) über die zugehörige Schnittstelle der Steuereinrichtung (3) mit dieser kommuniziert;
wobei in der Speichereinheit des Kits (2)
- ein oder mehrere elektronisch gespeicherte Messprotokolle, umfassend Instruktionen für die Durchführung der Analyse der biologischen Probe (14) mittels der Marker; und
- elektronisch gespeicherte Software zur Auswertung von Messergebnissen, die von der biologischen Probe (14) mittels der Marker und unter Verwendung der Messprotokolle erhalten wurden
gespeichert sind.

2. Analysevorrichtung nach Anspruch 1,
**dadurch gekennzeichnet,**
**dass** sie ferner zumindest eine der folgenden Einheiten umfasst:
- eine Datenbank (12) zum Speichern von Bilddaten und/oder Analysedaten, wobei die Datenbank (12) über die zugehörige Schnittstelle der Steuereinrichtung (3) mit dieser kommuniziert; und
- eine Visualisierungseinheit (11) zur Visualisierung von Bilddaten und/oder Analysedaten, wobei die Visualisierungseinheit (11) über die zugehörige Schnittstelle der Steuereinrichtung (3) mit dieser kommuniziert.

3. Analysevorrichtung nach einem der vorstehenden Ansprüche,
**dadurch gekennzeichnet,**
**dass** die Speichereinheit des Kits (2) eine Schnittstelle aufweist, die die Kommunikation der Einheit zur elektronischen Speicherung mit einer anderen elektronischen Einheit ermöglicht.

4. Analysevorrichtung nach einem der vorstehenden Ansprüche,
**dadurch gekennzeichnet,**
**dass** sie die Steuereinrichtung (3) ferner eine oder mehrere der folgenden Schnittstellen umfasst:
- eine Schnittstelle, die die Kommunikation zwischen der Datenverarbeitungseinheit (13) und einer Datenbank (12) zum Speichern von Bilddaten und/oder Analysedaten ermöglicht; und
- eine Schnittstelle, die die Kommunikation zwischen der Datenverarbeitungseinheit (13) und einer Visualisierungseinheit (11) zur Visualisierung von Bilddaten und/oder Analysedaten ermöglicht.

5. Verfahren zur automatisierten Analyse einer biologischen Probe (14) mittels einer Vorrichtung (1) gemäß einem der Ansprüche 1 bis 4, umfassend die Schritte:
(a) Bereitstellen eines Kits (2), umfassend die zur Analyse einer vorgegebenen biologischen Probe (14) erforderlichen Marker, Betriebsstoffe, Messprotokolle und optional Software;
(b) Einführen des Kits (2) in die Steuereinrichtung (3) und Übertragen von dort gespeicherten Messprotokollen in die Datenverarbeitungseinheit (13) der Steuereinrichtung (3);
(c) Bestimmen der Anweisungen aus den Messprotokollen, die zur Steuerung der Transfereinheit (8) erforderlich sind, mittels der Datenverarbeitungseinheit (13) und Übertragen dieser Anweisungen über die zugehörige Schnittstelle der Steuereinrichtung (3) auf die Transfereinheit (8);
(d) Bestimmen der Anweisungen aus den Messprotokollen, die zur Steuerung der Detektionseinheit (9) erforderlich sind, mittels der Datenverarbeitungseinheit (13) und Übertragen dieser Anweisungen über die zugehörige Schnittstelle der Steuereinrichtung (3) auf die Detektionseinheit (9);
(e) Transfer von Markern und Betriebsstoffen von dem Kit (2) zu der Detektionseinheit (9) mittels der Transfereinheit (8) anhand der in Schritt (c) übertragenen Anweisungen;
(f) Erzeugen von Bilddaten der biologischen Probe (14) mittels der Detektionseinheit (9) anhand der in Schritt (d) übertragenen Anweisungen;
(g) Bestimmen der Anweisungen aus den Messprotokollen, die zur Steuerung der Analyseeinheit (10) erforderlich sind, mittels der Datenverarbeitungseinheit (13) und Übertragen dieser Anweisungen über die zugehörige Schnittstelle der Steuereinrichtung (3) zu der Analyseeinheit (10), wobei Software, die vom Kit (2) in die Datenverarbeitungseinheit (13) übertragen worden ist, von der Datenverarbeitungseinheit (13) zu der Analyseeinheit (10) übertragen wird; und
(h) Transfer der in Schritt (f) erhaltenen Bilddaten zu der Analyseeinheit (10) und Analysieren der Bilddaten mittels der Analyseeinheit (10) unter Verwendung der in Schritt (e) übertragenen Anweisungen und der in Schritt (g) übertragenen Software, wodurch Analysedaten erhalten werden.

6. Verfahren nach Anspruch 5,
**dadurch gekennzeichnet,**
**dass** die Bilddaten und/oder die Analysedaten zu der Datenverarbeitungseinheit (13) übertragen und dort in einer Datenbank (12) gespeichert werden.

7. Verfahren nach Anspruch 5 oder Anspruch 6,
**dadurch gekennzeichnet,**
**dass** die Bilddaten und/oder Analysedaten von der Datenverarbeitungseinheit (13) über deren zugehörige Schnittstelle zu einer Visualisierungseinheit (11) übertragen werden.

## Claims

1. An analyzer (1) for the automated analysis of a biological specimen (14) that comprises
- a kit (2) comprising a unit (receiving unit) with a number of reservoirs (5) for receiving one or more marker(s) required for the analysis of the biological specimen (14) as well as, if needed, the process aids required therefore as well as a unit (memory unit) for the electronic storage of one or more electronically stored test record(s) comprising instructions for the performance of the analysis of the biological specimen (14) by means of the markers, wherein the memory unit is integrated within the receiving unit and wherein the receiving unit of the kit (2) is realized as a microfluidics system;
- a control system (3) comprising
- a data processing unit (13);
- a unit for receiving the kit (2);
- an interface allowing the communication between the data processing unit (13) and the kit (2);
- an interface allowing the communication between the data processing unit (13) and a transfer unit (8) for the transfer of the markers and, if present, process aids from the kit (2) to a detection unit (9);
- an interface allowing the communication between the data processing unit (13) and the detection unit (9) for the generation of image data of the biological specimen (14); and
- an interface allowing the communication between the data processing unit (13) and an analysis unit (10) to analyze the image data obtained by means of the detection unit (9) to generate analytical data;
- a transfer unit (8) for the transfer of the markers from the kit (2) to a detection unit (9), wherein the transfer unit (8) communicates with the control system (3) via its related interface and the transfer unit (8) is realized as a microfluidics system;
- a detection unit (9) to generate image data of the biological specimen (14), wherein the detection unit (9) communicates with the control system (3) via its related interface; and
- an analysis unit (10) to analyze the image data obtained by means of the detection unit (9) to generate analytical data, wherein the analysis unit (10) communicates with the control system (3) via its related interface;
wherein in the memory unit of the kit (2)
- one or more electronically stored test record(s) comprising instructions for performing the analysis of the biological specimen (14) by means of the markers; and
- electronically stored software for the evaluation of measuring results obtained from the biological specimen (14) by means of the markers and by using the test records
are stored.

2. The analyzer according to claim 1,
**characterized in that**
it further comprises at least one of the following units:
- a data base (12) to store image data and/or analytical data, wherein the data base (12) communicates with the control system (3) via its related interface; and
- a visualization unit (11) to visualize image data and/or analytical data, wherein the visualization unit (11) communicates with the control system (3) via its related interface.

3. The analyzer according to any one of the preceding claims,
**characterized in that**
the memory unit of the kit (2) has an interface allowing the communication of the unit for electronic storage with another electronic unit.

4. The analyzer according to any one of the preceding claims,
**characterized in that**
the control system (3) further comprises one or more of the following interfaces:
- an interface allowing the communication between the data processing unit (13) and a data base (12) for the storage of image data and/or analytical data; and
- an interface allowing the communication between the data processing unit (13) and a visualization unit (11) for the visualization of image data and/or analytical data.

5. A method for the automated analysis of a biological specimen (14) by means of a device (1) according to any one of claims 1 to 4 that comprises the steps of:
(a) providing a kit (2) comprising the markers, process aids, test records, and optionally software that are required for the analysis of a given biological specimen (14);
(b) introducing the kit (2) into the control system (3) and transferring test records stored there into the data processing unit (13) of the control system (3);
(c) determining the instructions from the test records required for controlling the transfer unit (8) by means of the data processing unit (13) and transferring said instructions via the related interface of the control system (3) to the transfer unit (8);
(d) determining the instructions from the test records required for controlling the detection unit (9) by means of the data processing unit (13) and transferring said instructions via the related interface of the control system (3) to the detection unit (9);
(e) transferring markers and process aids from the kit (2) to the detection unit (9) by means of the transfer unit (8) on the basis of the instructions transferred in step (c);
(f) generating image data of the biological specimen (14) by means of the detection unit (9) on the basis of the instructions transferred in step (d);
(g) determining the instructions from the test records required for controlling the analysis unit (10) by means of the data processing unit (13) and transferring said instructions via the related interface of the control system (3) to the analysis unit (10), wherein software that has been transferred from the kit (2) into the data processing unit (13) is transferred from the data processing unit (13) to the analysis unit (10); and
(h) transferring the image data obtained in step (f) to the analysis unit (10) and analyzing the image data by means of the analysis unit (10) using the instructions transferred in step (e) and the software transferred in step (g) to obtain analytical data.

6. The method according to claim 5,
**characterized in that**
the image data and/or analytical data are transferred to the data processing unit (13) and there are stored in the data base (12).

7. The method according to claim 5 or claim 6,
**characterized in that**
the image data and/or analytical data are transferred from the data processing unit (13) via its related interface to a visualization unit (11).

## Revendications

1. Dispositif d'analyse (1) pour l'analyse automatique d'un échantillon biologique (14), comprenant
- un kit (2), comprenant une unité (unité réceptrice) avec un grand nombre de réservoirs (5) pour recevoir un ou plusieurs marqueurs qui sont nécessaires pour l'analyse de l'échantillon biologique (14), ainsi que, si c'est nécessaire, les produits nécessaires au fonctionnement, ainsi qu'une unité (unité de mémoire) pour la sauvegarde électronique d'un ou de plusieurs protocoles de mesure sauvegardés électroniquement, comprenant des instructions pour l'exécution de l'analyse de l'échantillon biologique (14) au moyen des marqueurs, étant donné que l'unité de mémoire est intégrée dans l'unité réceptrice et que l'unité réceptrice du kit (2) est réalisée en tant que système microfluidique;
- un dispositif de commande (3), comprenant
- une unité de traitement des données (13);
- une unité de réception du kit (2);
- une interface qui permet la communication entre l'unité de traitement des données (13) et le kit (2);
- une interface qui permet la communication entre l'unité de traitement des données (13) et une unité de transfert (8) pour le transfert des marqueurs et, s'il y en a, des produits de fonctionnement du kit (2) vers une unité de détection (9);
- une interface qui permet la communication entre l'unité de traitement des données (13) et l'unité de détection (9) pour la génération de données-images de l'échantillon biologique (14); et
- une interface qui permet la communication entre l'unité de traitement des données (13) et une unité d'analyse (10) pour l'analyse des données-images obtenues au moyen de l'unité de détection (9) par génération de données d'analyse;
- une unité de transfert (8) pour le transfert des marqueurs du kit (2) vers une unité de détection (9), l'unité de transfert (8) communiquant avec le dispositif de commande (3) par l'interface qui lui est attribuée et l'unité de transfert (8) étant réalisée en tant que système microfluidique;
- une unité de détection (9) pour la génération de données-images de l'échantillon biologique (14), l'unité de détection (9) communiquant avec le dispositif de commande (3) par l'interface qui lui est attribuée; et
- une unité d'analyse (10) pour l'analyse des données-images obtenues au moyen de l'unité de détection (9) par génération de données d'analyse, l'unité d'analyse (10) communiquant avec l'unité de commande (3) par l'interface qui lui est attribuée;
étant donné que dans l'unité de mémoire du kit (2) sont sauvegardés
- un ou plusieurs protocoles sauvegardés électroniquement, comprenant des instructions pour l'exécution de l'analyse de l'échantillon biologique (14) au moyen des marqueurs; et
- des logiciels sauvegardés électroniquement pour l'interprétation des résultats de mesure qui ont été obtenus de l'échantillon biologique (14) au moyen des marqueurs et en utilisant les protocoles de mesure.

2. Dispositif d'analyse selon la revendication 1,
**caractérisé par le fait**
**qu'**il comprend en outre au moins une des unités suivantes:
- une banque de données (12) pour la sauvegarde de données-images et/ou de données d'analyse, la banque de données (12) communiquant avec le dispositif de commande (3) par l'interface qui lui est attribué; et
- une unité de visualisation (11) pour la visualisation de données-images et/ou de données d'analyse, l'unité de visualisation (11) communiquant avec le dispositif de commande (3) par l'interface qui lui est attribué.

3. Dispositif d'analyse selon une des revendications précédentes,
**caractérisé par le fait**
**que** l'unité de mémoire du kit (2) présente une interface qui permet la communication de l'unité pour la sauvegarde électronique avec une autre unité électronique.

4. Dispositif d'analyse selon une des revendications précédentes,
**caractérisé par le fait**
**que** le dispositif de commande (3) comprend en outre une ou plusieurs des interfaces suivantes:
- une interface qui permet la communication entre l'unité de traitement des données (13) et une banque de données (12) pour la sauvegarde de donnés-images et/ou des données d'analyse; et
- une interface qui permet la communication entre l'unité de traitement des données (13) et une unité de visualisation (11) pour la visualisation de données-images et/ou de données d'analyse.

5. Procédé pour l'analyse automatisée d'un échantillon biologique (14) au moyen d'un dispositif (1) selon une des revendications 1 à 4, comprenant les étapes:
(a) disposition d'un kit (2), comprenant marqueurs, produits de fonctionnement, protocoles de mesure et logiciel, celui-ci en option, nécessaires pour l'analyse d'un échantillon biologique (14) donné;
(b) introduction du kit (2) dans le dispositif de commande (3) et report des protocoles de mesure, qui y sont conservés, dans l'unité de traitement des données (13) du dispositif de commande (3);
(c) déterminer les instructions, contenues dans les protocoles de mesure, qui sont nécessaires pour la commande de l'unité de transfert (8), au moyen de l'unité de traitement des données (13) et report de ces instructions à l'unité de transfert (8) par l'interface attribuée à l'unité de commande (3);
(d) déterminer les instructions, contenues dans les protocoles de mesure, qui sont nécessaires pour la commande de l'unité de détection (9), au moyen de l'unité de traitement des données (13) et report de ces instructions sur l'unité de détection (9) par l'interface attribuée à l'unité de commande (3):
(e) transfert de marqueurs et de produits de fonctionnement du kit (2) vers l'unité de détection (9) au moyen de l'unité de transfert (8) en suivant les instructions reportées dans l'étape (c);
(f) génération de données-images de l'échantillon biologique (14) au moyen de l'unité de détection (9) en suivant les instructions reportées dans l'étape (d);
(g) déterminer les instructions des protocoles de mesure, qui sont requises pour la commande de l'unité d'analyse (10), au moyen de l'unité de traitement des données (13), et report de ces instructions vers l'unité d'analyse (10) par l'interface attribuée au dispositif de commande (3), le logiciel qui été reporté du kit (2) dans l'unité de traitement des données (13) étant lui-même reporté de l'unité de traitement des données (13) vers l'unité d'analyse (10); et
(h) transfert des données-images obtenues dans l'étape (f) vers l'unité d'analyse (10) et analyse des données-images au moyen de l'unité d'analyse (10) en se servant des instructions reportées dans l'étape (e) et du logiciel reporté dans l'étape (g), ce qui permet d'obtenir les données d'analyse.

6. Procédé selon la revendication 5,
**caractérisé par le fait**
**que** les données-images et/ou les données d'analyse sont transférées vers l'unité de traitement des données (13) et y sont sauvegardées dans une banque de données (12).

7. Procédé selon la revendication 5 ou la revendication 6,
**caractérisé par le fait**
**que** les données-images et/ou les données d'analyse sont transférées de l'unité de traitement des données (13), par l'interface attribuée à celle-ci, vers une unité de visualisation (11).
